# EUROPEAN PATENT APPLICATION

(11) **EP 4 818 325 A1**
(43) Date of publication of application: **07.10.2026**
(21) Application number: 24897005.5
(22) Date of filing: 22.08.2024
(51) Int. Cl.: C12P 7/56, C12P 7/46, C12P 7/54

(54) **METHOD FOR PRODUCING ORGANIC COMPOUND**

(30) Priority: 28.11.2023 JP 2023201140
(71) Applicant: The University of Osaka, Suita-shi, Osaka 565-0871 (JP)
(72) Inventor: NAKANISHI, Shuji, Suita-shi, Osaka 565-0871 (JP); TABATA, Hiro, Suita-shi, Osaka 565-0871 (JP); NISHIJIMA, Hiroaki, Suita-shi, Osaka 565-0871 (JP); YAMADA, Yuki, Suita-shi, Osaka 565-0871 (JP)
(74) Representative: Müller-Boré & Partner Patentanwälte PartG mbB
(86) International application number: PCT/JP2024/029914
(87) International publication number: WO 2025/115313

(57) **Abstract**

The purpose of the present invention is to provide a technique for producing a useful substance by using microorganisms without competing with a food supply and without consuming large amounts of finite resources. A method for producing an organic compound, the method comprising a substance production step for, in a sugar-containing liquid containing a non-natural sugar, causing microorganisms to use the non-natural sugar as a substrate to produce a substance.

## Description

### Technical Field

The present invention relates to a technique for producing an organic compound using a chemically synthesized sugar as a starting material. More specifically, the present invention relates to a technique for causing a microorganism to use a sugar-containing liquid containing a non-natural sugar as a substrate to produce an organic compound.

### Background Art

Crop-derived sugars are the most typical substrates to be used for microbial production of useful substances. Such bio-production processes have been shown to be environmentally friendly with lower greenhouse gas emissions than conventional petrochemical processes (NPL 1). However, the biomass production capacity of the earth is limited by biophysical boundaries and cannot meet the enormous demand for fuel and chemical production (NPL 2). In recent years, although the use of biomass is limited more as compared to fossil fuels, about 13% of the world's farmlands are used for the production of biofuels and fibers (NPL 3). Therefore, the expansion of the use of industrial biomass may cause a problem of competition with food demand (NPL 3). In addition, industrial agriculture to meet such enormous demands has negative aspects such as enormous lands, large-scale consumption of depleting resources such as water, nitrogen and phosphorus, water pollution due to eutrophication, and loss of biological diversity (NPLs 3 and 5). Actually, there is also an indication that human material requirements in agriculture may be a factor beyond the boundaries of the planet. (NPL 6).

On the other hand, a formose reaction, which is a method for catalytically chemically synthesizing a saccharide from formaldehyde, is known, and is attracting attention as an upgrading technique for carbon dioxide and formaldehyde. In recent years, identification of formose reaction products (NPL 7), selection of an appropriate catalyst and appropriate reaction conditions (NPL 8), and chemical kinetics (NPL 9) have progressed, and reaction selectivity has been improved. Formose reaction products contain branched sugar chains and the like that do not exist in nature (NPL 10), and basically have toxicity to organisms. Actually, an example in which a rat fed with a feed containing 50% of a synthetic sugar liquid died has also been reported (NPL 11).

### Citation List

### Non Patent Literature

NPL 1: W. J. Groot, T. Boren, The International Journal of Life Cycle Assessment 2010, 15, 970-984
NPL 2: K. H. Erb, C. Lauk, T. Kastner, A. Mayer, M. C. Theurl, H. Haberl, Nat Commun 2016, 7, 11382
NPL 3: J. Poore, T. Nemecek, Science 2018, 360, 987-992.
NPL 4: A. Muscat, E. M. de Olde, I. J. M. de Boer, R. Ripoll-Bosch, Global Food Security 2020, 25.
NPL 5: Y. Lu, S. Song, R. Wang, Z. Liu, J. Meng, A. J. Sweetman, A. Jenkins, R. C. Ferrier, H. Li, W. Luo, T. Wang, Environ Int 2015, 77, 5-15
NPL 6: J. A. Foley, N. Ramankutty, K. A. Brauman, E. S. Cassidy, J. S. Gerber, M. Johnston, N. D. Mueller, C. O'Connell, D. K. Ray, P. C. West, C. Balzer, E. M. Bennett, S. R. Carpenter, J. Hill, C. Monfreda, S. Polasky, J. Rockstrom, J. Sheehan, S. Siebert, D. Tilman, D. P. Zaks, Nature 2011, 478, 337-342.
NPL 7: W. E. Robinson, E. Daines, P. van Duppen, T. de Jong, W. T. S. Huck, Nat. Chem. 2022, 14, 623-631
NPL 8: J. B. Lambert, S. A. Gurusamy-Thangavelu, K. Ma, Science 2010, 327, 984-986.
NPL 9: P. van Duppen, E. Daines, W. E. Robinson, W. T. S. Huck, J. Am. Chem. Soc. 2023, 145, 7559-7568.
NPL 10: R. D. Partridge, A. H. Weiss, D. Todd, Carbohydr. Res. 1972, 24, 29-44.
NPL 11: T. Mizuno, A. H. Weiss, Adv. Carbohydr. Chem. Biochem. 1974, 29, 173-227

### Summary of Invention

### Technical Problem

To sustainably realize the production of useful substances using microorganisms, it is necessary to address the problem regarding the supply of biomass-derived sugars as a substrate (that is, competition with food supply and large-scale consumption of finite resources) as described above. Although the formose reaction product is also a sugar mixture, it is recognized that the biomass-derived sugars cannot be essentially substituted thereby because the biomass-derived sugars are composed of natural sugars whereas the formose reaction product contains a non-natural sugar and has biological toxicity.

Accordingly, the purpose of the present invention is to provide a technique for producing a useful substance by using microorganisms without competing with a food supply and without massively consuming finite resources.

### Solution to Problem

The present inventors have found that a formose reaction product, which should not be able to substitute for biomass-derived sugars, can unexpectedly be used as a substrate for substance production using a microorganism, and as a result, it has been found that an organic compound can be produced in a microorganism by using a non-natural sugar that does not compete with a food supply and does not require large-scale consumption of finite resources as a substrate.

In summary, the present invention provides aspects of invention as itemized below.
Item 1. A method for producing an organic compound, the method comprising a substance production step for, in a sugar-containing liquid containing a non-natural sugar, causing a microorganism to produce a substance by using the non-natural sugar as a substrate.
Item 2. The production method according to item 1, further comprising a step of cultivation of the microorganism before the substance production step.
Item 3. The production method according to item 1, wherein a concentration of the microorganism in the sugar-containing liquid is 0.5 to 25 w/v%.
Item 4. The production method according to any one of items 1 to 3, wherein the non-natural sugar comprises at least one among a triose through a hexose.
Item 5. The production method according to any one of items 1 to 4, wherein the non-natural sugar is selected from the group consisting of stereoisomers of 1,3,4,5-tetrahydroxypentan-2-one, 1,3,4-trihydroxy-3-(hydroxymethyl)butan-2-one, and 1,2,4,5,6-pentahydroxyhexan-3-one.
Item 6. The production method according to any one of items 1 to 5, wherein the sugar-containing liquid contains at least one among formaldehyde (C1a), glycolaldehyde (C2a), glyceraldehyde (C3a), dihydroxyacetone (C3k), erythrulose (C4k), and erythrose (C4a) and threose (C4a).
Item 7. The production method according to any one of items 1 to 6, wherein the sugar-containing liquid contains erythrulose (C4k) at an amount to be more than 0 mM and not more than 20 mM.
Item 8. The production method according to any one of items 1 to 7, wherein the sugar-containing liquid contains erythrose and/or threose (C4a) at an amount to be more than 0 mM and not more than 25 mM in total.
Item 9. The production method according to any one of items 1 to 8, wherein the sugar-containing liquid contains formaldehyde (C1a) at an amount to be more than 0 mM and not more than 0.6 mM, glycolaldehyde (C2a) at an amount to be more than 0 mM and not more than 4 mM, glyceraldehyde (C3a) at an amount to be more than 0 mM and not more than 7 mM, and/or dihydroxyacetone (C3k) at an amount to be more than 0 mM and not more than 4 mM.
Item 10. The production method according to any one of items 1 to 9, wherein the sugar-containing liquid is a formose reaction product.
Item 11. The production method according to any one of items 1 to 9, wherein the sugar-containing liquid is a base-treated product of a formose reaction product.
Item 12. The production method according to any one of items 1 to 11, wherein the sugar in the sugar-containing liquid is contained at an amount of 0.1 to 60 w/v% in total.
Item 13. The production method according to any one of items 1 to 12, wherein the organic compound comprises lactic acid and/or a salt thereof.
Item 14. The production method according to any one of items 1 to 13, wherein the organic compound comprises acetic acid, succinic acid, fumaric acid salt, and/or salts thereof.
Item 15. The production method according to any one of items 1 to 14, wherein the organic compound comprises β-glucan.
Item 16. The production method according to any one of items 1 to 15, wherein the microorganism is selected from the group consisting of bacteria, yeast, and microalgae.

### Advantageous Effects of Invention

According to the present invention, it becomes possible to produce a useful substance by using microorganisms without competing with a food supply and without needing large-scale consumption of finite resources.

### Brief Description of Drawings

[Fig. 1] Fig. 1 shows a photograph of a synthetic sugar liquid as a formose reaction product, an HPLC chromatogram of the formose reaction product, and identification results.
[Fig. 2] Fig. 2(a) shows the changes in OD₆₀₀ with time when C. glutamicum was cultivated in an inorganic medium containing a synthetic sugar liquid. N = 3, and each error bar indicates a 95% confidence interval. Fig. 2(b) shows an HPLC chromatogram of a supernatant before cultivation (before cultivation) and an HPLC chromatogram of a supernatant after cultivation for 57 hours in a medium containing 0.5% (w/v) of sugar (after cultivation).
[Fig. 3a] Fig. 3a shows the changes in OD₆₀₀ with time when C1a was added at various concentrations to an inorganic medium containing 0.1% of glucose. N = 3, and each error bar indicates a 95% confidence interval.
[Fig. 3b] Fig. 3b shows the changes in OD₆₀₀ with time when C2a was added at various concentrations to an inorganic medium containing 0.1% of glucose. N = 3, and each error bar indicates a 95% confidence interval.
[Fig. 3c] Fig. 3c shows the changes in OD₆₀₀ with time when C3a was added at various concentrations to an inorganic medium containing 0.1% of glucose. N = 3, and each error bar indicates a 95% confidence interval.
[Fig. 3d] Fig. 3d shows the changes in OD₆₀₀ with time when C3k was added at various concentrations to an inorganic medium containing 0.1% of glucose. N = 3, and each error bar indicates a 95% confidence interval.
[Fig. 3e] Fig. 3e shows the changes in OD₆₀₀ with time when C4a was added at various concentrations to an inorganic medium containing 0.1% of glucose. N = 3, and each error bar indicates a 95% confidence interval.
[Fig. 3f] Fig. 3f shows the changes in OD₆₀₀ with time when C4k was added at various concentrations to an inorganic medium containing 0.1% of glucose. N = 3, and each error bar indicates a 95% confidence interval.
[Fig. 4] Fig. 4 shows results of comparison of the changes with time of OD₆₀₀ between the case of adding 5% of a synthetic sugar liquid to an inorganic medium (Syn-sugar 5%) and the case of adding a base-treated synthetic sugar liquid to an inorganic medium (Base treatment). The result of Base treatment was normalized on the basis of the final growth value of a control experiment (glucose 0.1%) performed in the same batch for comparison with the value of Syn-sugar 5%. N = 3, and each error bar indicates a 95% confidence interval.

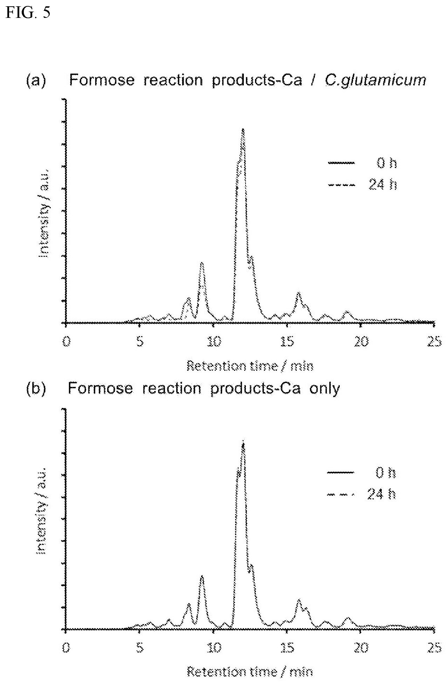

[Fig. 5] Fig. 5(a) shows HPLC chromatograms produced by analyzing C5-C6 sugars in the supernatants collected immediately after (0 h) and 24 hours after (24 h) lactic acid fermentation under the condition where 1% (w/v) of a synthetic sugar (formose reaction product-Ca) was added in the presence of C. glutamicum. Fig. 5(b) shows HPLC chromatograms produced by analyzing C5-C6 sugars in the supernatants collected immediately after (0 h) and 24 hours after (24 h) lactic acid fermentation under the condition where 1% (w/v) of a synthetic sugar (formose reaction product-Ca) was added in the absence of C. glutamicum.
[Fig. 6] Fig. 6(a) shows HPLC chromatograms of the supernatants collected 24 hours after the start of lactic acid fermentation in the presence of C. glutamicum in the case of adding 1% (w/v) of a synthetic sugar (formose reaction product-Ca) (w/syn-sugar) and in the case of adding no sugar (w/o sugar). Fig. 6(b) shows the result of determination of the amount of an organic acid after reaction based on the HPLC analysis of (a). N = 3, and each error bar indicates a 95% confidence interval.
[Fig. 7] Fig. 7 shows, in the same manner as in Fig. 6(b), the result of determination of the amount of an organic acid obtained by lactic acid fermentation using 1% (w/v) of a synthetic sugar (formose reaction product-W) at different concentrations in the presence of C. glutamicum.
[Fig. 8] Fig. 8 shows HPLC chromatograms produced by analyzing the C5-C6 sugars in the supernatants collected immediately after (0 h) and 24 hours after (24 h) the start of reaction under the condition where 1% (w/v) of a synthetic sugar (formose reaction product-Ca) was added in the presence of E. Coli.
[Fig. 9] Fig. 9 shows HPLC chromatograms produced by analyzing the C5-C6 sugars in the supernatants collected immediately after (0 h) and 24 hours after (24 h) the start of reaction under the condition where 1% (w/v) of a synthetic sugar (formose reaction product-W) was added in the presence of E. Coli.
[Fig. 10] Fig. 10 shows HPLC chromatograms of the supernatants collected 24 hours after the start of reaction in the presence of E. Coli in the case of adding 1% (w/v) of a synthetic sugar (formose reaction product-Ca) (w/syn-sugar) and in the case of adding no sugar (w/o sugar).
[Fig. 11] Fig. 11 shows HPLC chromatograms of the supernatants collected 24 hours after the start of reaction in the presence of E. Coli in the case of adding 1% (w/v) of a synthetic sugar (formose reaction product-W) (w/syn-sugar) and in the case of adding no sugar (w/o sugar).
[Fig. 12] Fig. 12 shows HPLC chromatograms produced by analyzing the C5-C6 sugars in the supernatants collected immediately after (0 h) and 24 hours after (24 h) the start of reaction under the condition where 1% (w/v) of a synthetic sugar (formose reaction product-Ca) was added in the presence of S. cerevisiae.
[Fig. 13] Fig. 13 shows HPLC chromatograms produced by analyzing the C5-C6 sugars in the supernatants collected immediately after (0 h) and 24 hours after (24 h) the start of reaction under the condition where 1% (w/v) of a synthetic sugar (formose reaction product-W) was added in the presence of S. cerevisiae.
[Fig. 14] Fig. 14 shows HPLC chromatograms of the supernatants collected 24 hours after the start of reaction in the presence of S. cerevisiae in the case of adding 1% (w/v) of a synthetic sugar (formose reaction product-Ca) (w/syn-sugar) and in the case of adding no sugar (w/o sugar).
[Fig. 15] Fig. 15 shows HPLC chromatograms of the supernatants collected 24 hours after the start of reaction in the presence of S. cerevisiae in the case of adding 1% (w/v) of a synthetic sugar (formose reaction product-W) (w/syn-sugar) and in the case of adding no sugar (w/o sugar).

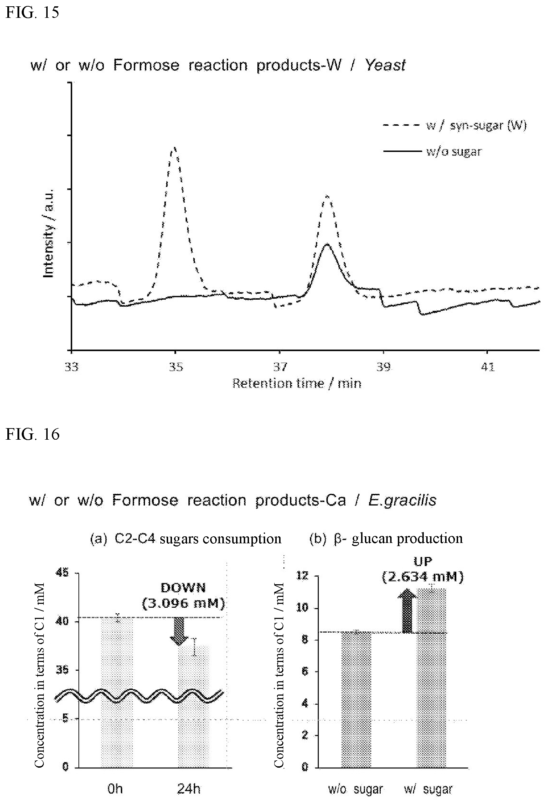

[Fig. 16] Fig. 16(a) shows the concentrations in terms of C1 sugar of C2-C4 sugars contained in the supernatants collected immediately after (0 h) and 24 hours after (24 h) the start of reaction under the condition where 1 w/v% of a synthetic sugar (formose reaction product-Ca) was added in the presence of E.gracilis. Fig. 16(b) shows the concentrations in terms of C1 sugar of β-glucan contained in the supernatants collected 24 hours after the start of reaction in the presence of E.gracilis in the case of adding 1% (w/v) of a synthetic sugar (formose reaction product-Ca) (w/ sugar) and in the case of adding no sugar (w/o sugar).

### Description of Embodiments

The method for producing an organic compound of the present invention is characterized by comprising a substance production step for, in a sugar-containing liquid containing a non-natural sugar, causing a microorganism to produce a substance by using the non-natural sugar as a substrate. Hereinafter, the method for producing a sugar utilization product of the present invention will be described in detail. In the present description, a sugar having N carbon atoms (N is an integer of 2 or more) is denoted by CN (N-carbon sugar), and particularly, aldose and ketose are denoted by CNa and CNk, respectively. In addition, as to substances other than sugars, formaldehyde is also denoted by C1a.

### 1. Substance production step

### 1-1. Sugar-containing liquid

### 1-1-1. Sugar contained in sugar-containing liquid

The sugar contained in the sugar-containing liquid containing a non-natural sugar is not particularly limited, and examples thereof include C2 sugars, C3 sugars, C4 sugars, C5 sugars, C6 sugars, C7 sugars, and C8 sugars, and any one of these sugars may be contained, or two or more of these sugars may be contained in combination.

Sugar-containing liquids containing non-natural sugars typically contain sugar products obtained by chemical synthesis. Such sugar products obtained by chemical synthesis include many known compounds, and in the present invention, any sugar product containing a known compound and containing a non-natural sugar can be used without particular limitation. Preferred examples of the sugar contained in the sugar product in the present invention include the compounds represented by the following (C2-1) and (C2-2) as C2 sugars; the compounds represented by the following (C3-1) to (C3-3) as C3 sugars; the compounds represented by the following (C4-1) to (C4-5) as preferred C4 sugars; compounds represented by the following (C5-1) to (C5-9) as C5 sugars; and the compounds represented by the following (C6-1) to (C6-18) as C6 sugars. The following compounds include any stereoisomers (for example, enantiomers (D-sugar, L-sugar) and racemates thereof).

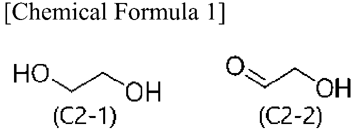

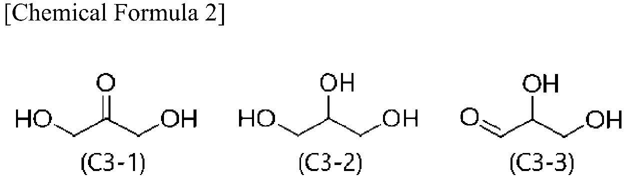

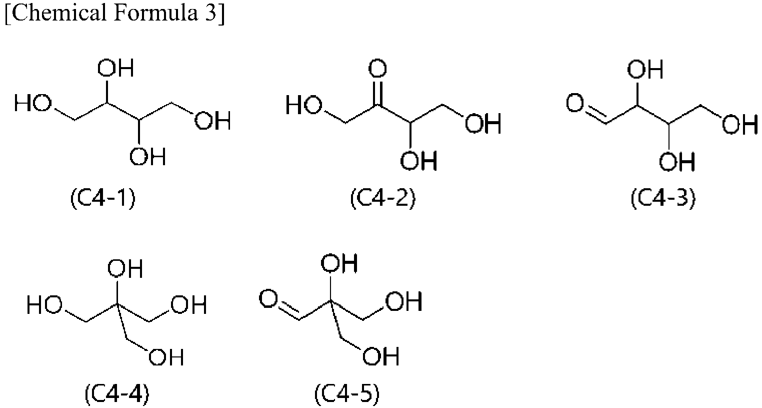

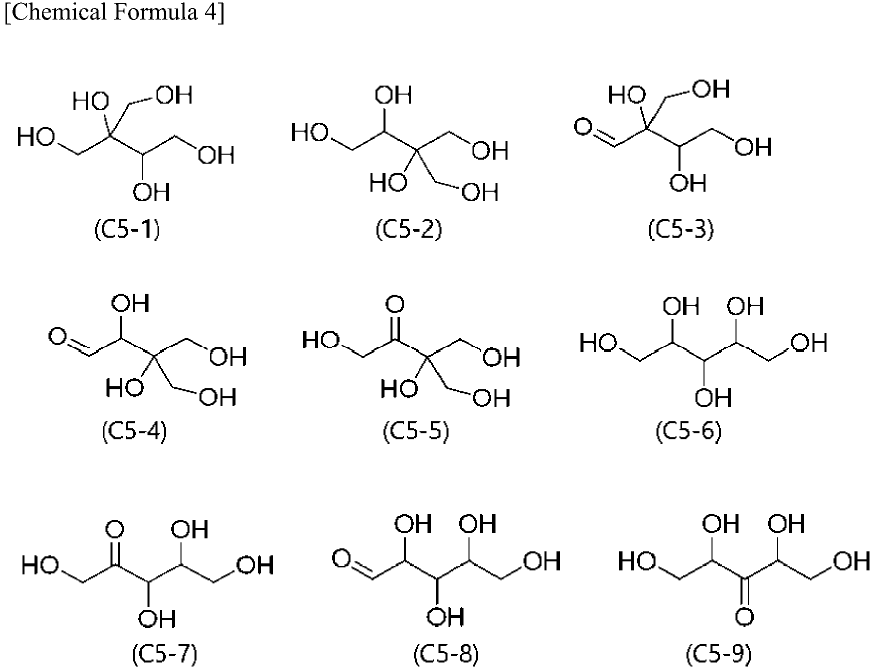

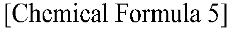

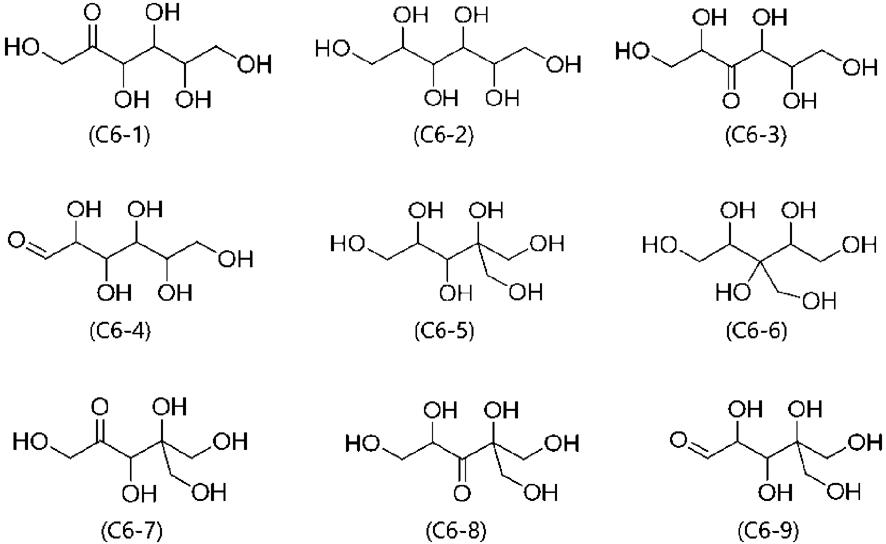

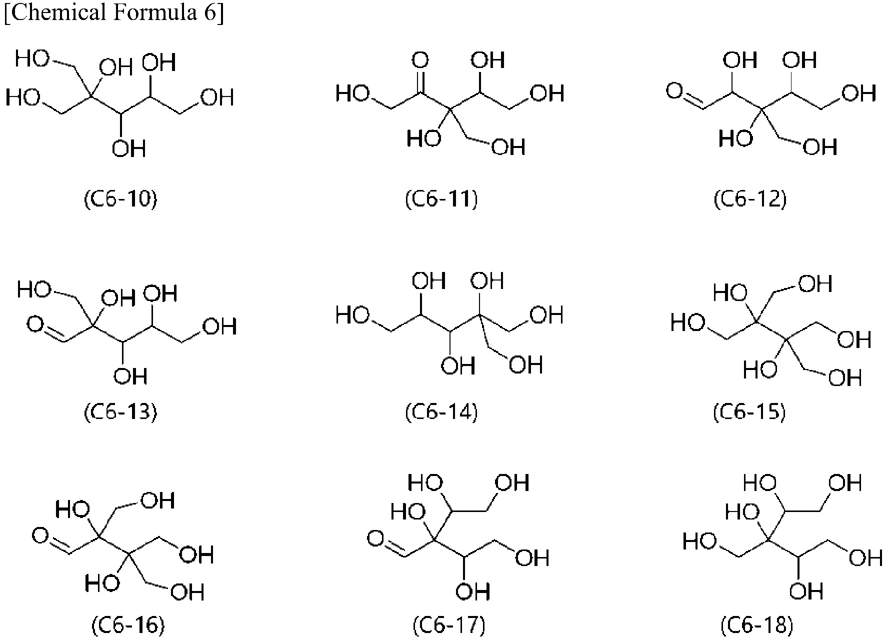

Examples of the non-natural sugar preferably include at least one among a C3 sugar, a C4 sugar, a C5 sugar, and a C6 sugar, more preferably include a C5 sugar and/or a C6 sugar, and further preferably include a linear C5 sugar such as 1,3,4,5-tetrahydroxypentan-2-one (the compound represented by the above (C5-7)), a branched C5 sugar such as 1,3,4-trihydroxy-3-(hydroxymethyl)butan-2-one (the compound represented by the above (C5-5)), a branched C6 sugar such as 1,2,4,5,6-pentahydroxyhexan-3-one (the compound represented by the above (C6-3)), and any stereoisomers thereof. Examples of the non-natural sugar also include L-sugars. These non-natural sugars may be used singly, or two or more of them may be used in combination. In the present invention, at least one of these non-natural sugars can be used as a substrate for substance production.

The sugar-containing liquid may contain a natural sugar in addition to the non-natural sugar. The natural sugar is merely required to have the same structure as a sugar existing in nature, and may be either one derived from a natural product or one generated by chemical synthesis. Examples thereof include formaldehyde (C1a), glycolaldehyde (C2a, the compound represented by the above (C2-2)), glyceraldehyde (C3a, the compound represented by the above (C3-3)), dihydroxyacetone (C3k, the compound represented by the above (C3-1)), erythrose or threose (C4a, the compound represented by the above (C4-1)), erythrulose (C4k, the compound represented by the above (C4-2)), and glucose (the compound represented by (C6-4) above). These natural sugars may be used singly, or two or more of them may be used in combination. In the present invention, at least one of these natural sugars can also be used as a substrate for substance production.

### 1-1-2. Other components contained in sugar-containing liquid

The sugar-containing liquid may contain at least one among formaldehyde (C1a), sugar alcohols, and sugar carboxylic acids in addition to the sugar. As the sugar alcohols and sugar carboxylic acids, known sugar alcohols and sugar carboxylic acids contained in sugar products obtained by chemical synthesis (for example, those reported in Life 2020, 10(8), 125; https://doi.org/10.3390/life10080125) can be used.

### 1-1-3. Content of sugar

When the sugar-containing liquid contains at least one sugar among formaldehyde (C1a), glycolaldehyde (C2a), glyceraldehyde (C3a), dihydroxyacetone (C3k), erythrulose (C4k), and erythrose and threose (C4a), the concentrations of the sugars in the sugar-containing liquid are not particularly limited, but are preferably the concentrations shown below from the viewpoint of improving the growth environment of microorganisms.

· Formaldehyde (C1a): The concentration is preferably more than 0 mM and not more than 0.6 mM, more preferably more than 0 mM and not more than 0.5 mM, still more preferably more than 0 mM and not more than 0.4 mM, and further preferably more than 0 mM and not more than 0.2 mM or more than 0 mM and not more than 0.1 mM.
· Glycolaldehyde (C2a): The concentration is preferably more than 0 mM and not more than 4 mM, more preferably more than 0 mM and not more than 2 mM, still more preferably more than 0 mM and not more than 1 mM, and further preferably more than 0 mM and not more than 0.5 mM.
· Glyceraldehyde (C3a): The concentration is preferably more than 0 mM and not more than 7 mM, and more preferably more than 0 mM and not more than 6 mM, more than 0 mM and not more than 5 mM, more than 0 mM and not more than 2 mM, more than 0 mM and not more than 1 mM, or more than 0 mM and not more than 0.5 mM.
· Dihydroxyacetone (C3k): The concentration is preferably more than 0 mM and not more than 4 mM, more preferably more than 0 mM and not more than 3 mM, and still more preferably more than 0 mM and not more than 2 mM.
· Erythrulose (C4k): The concentration is preferably more than 0 mM and not more than 20 mM, more preferably more than 0 mM and not more than 20 mM, and still more preferably more than 0 mM and not more than 10 mM.
· Erythrose and/or threose (C4a): The concentration is, in total, preferably more than 0 mM and not more than 25 mM, more preferably more than 0 mM and not more than 20 mM, still more preferably more than 0 mM and not more than 15 mM, further preferably more than 0 mM and not more than 10 mM, and still further preferably more than 0 mM and not more than 5 mM.

The total amount of the sugars contained in the sugar-containing liquid is, for example, 0.1 to 60 w/v% or 0.5 to 10 w/v%, preferably 0.8 to 10 w/v%, and more preferably 1.8 to 10 w/v%, 1.8 to 6 w/v%, or 1.8 to 4 w/v%. The total amount of the sugars is a value derived by the method described in Examples on the basis of the total organic carbon (TOC) where all the sugars contained in the sugar-containing liquid are expressed by (CH₂O)ₙ (n is an integer of 2 or more).

### 1-1-4. Examples of sugar-containing liquid (sugar-containing liquid containing formose reaction product)

The sugar-containing liquid may be one obtained by any synthetic method as long as it contains a non-natural sugar. Preferably, the sugar-containing liquid may be a sugar-containing liquid containing a formose reaction product. The formose reaction method for obtaining a formose reaction product is known, and is not particularly limited as long as it is a method of non-enzymatically chemically synthesizing a saccharide from formaldehyde, and can be appropriately selected by those skilled in the art.

Typically, in a formose reaction, the reaction can be performed using formaldehyde as a substrate, and preferably using an appropriate initiator and an appropriate catalyst, under an appropriate liquid property condition.

The liquid property (pH) may be appropriately selected by those skilled in the art according to an initiator, a catalyst, and the like to be selected, and examples thereof include neutral (6.5 or more and 8.5 or less) and basic (more than 8.5).

Examples of the initiator include monosaccharides, disaccharides, oligosaccharides, and polysaccharides, and any one of these four forms of sugars may be used, or two or more of these forms may be used in combination. The monosaccharides may include diose, triose, tetrose, pentose, hexose, heptose, and the like, and these may be any of aldose and ketose, and may be any of those having a chain (linear or branched) structure and those having a cyclic structure. Examples of the monosaccharide include glycolaldehyde, dihydroxyacetone, glyceraldehyde, erythrose, threose, erythrulose, ribose, arabinose, xylose, lyxose, ribulose, xylulose, psicose, fructose, sorbose, tagatose, allose, altrose, glucose, mannose, gulose, idose, galactose, talose, and heptulose. Examples of the disaccharide include lactose, maltose, isomaltose, sucrose, and trehalose. Oligosaccharides are substances made up of 3 to 10 monosaccharide molecules condensed, and examples thereof include fructooligosaccharides, galactooligosaccharides, xylooligosaccharides, soybean oligosaccharides, isomaltooligosaccharides, lactosucrose, and raffinose. Polysaccharides are substances made up of 11 or more monosaccharide molecules condensed, and examples thereof include starch, dextrin, cellulose, glycogen, dextran, mutan, levan, and inulin. These initiators may be used singly, or two or more of them may be used in combination.

As a more specific example of the initiator, an intermediate product of a formose reaction can be selected, and more specific examples thereof include glycolaldehyde (C2a), glyceraldehyde (C3a), dihydroxyacetone (C3k), erythrulose (C4k), and erythrose and threose (C4a); one of these may be used singly, or two or more of them may be used in combination.

The catalyst may be either a homogeneous catalyst or a heterogeneous catalyst. Examples of the catalyst include alkali metal hydroxides (NaOH, KOH, etc.), zinc hydroxide (Zn(OH)₂), alumina, alkaline earth metal hydroxides (Ca(OH)₂, Mg(OH)₂, Ba(OH)₂, Sr(OH)₂, etc.), alkaline earth metal oxides (CaO, SrO, BaO, etc.), zeolite, and oxides, oxoacids, salts thereof (alkali metal salts (potassium salts, sodium salts, etc.), alkaline earth metal salts (calcium salts, barium salts, etc.), etc.), and complexes of Group 5 elements (V, Nb, Ta, Db, etc.) or Group 6 elements (Cr, Mo, W, Sg, etc.). Examples of the oxides, oxoacids, salts thereof, and complexes of the Group 5 elements or Group 6 elements include niobium oxide, molybdenum oxide, tungsten oxide, aluminum oxide, niobic acid and salts thereof, molybdic acid and salts thereof, tungstic acid and salts thereof, and aluminic acid and salts thereof. These catalysts may be used singly, or two or more of them may be used in combination.

Examples of the solvent include water and lower alcohols (for example, monohydric lower alcohols such as methanol, ethanol, isopropyl alcohol, and butanol), and these solvents may be used singly, or two or more of them may be used in combination.

As the formose reaction product, a product appropriately subjected to post-treatment may be used. As the post-treatment, a method of removing components other than the produced sugar (for example, the catalyst, the solvent, and the formaldehyde as a raw material) and/or a method of increasing the concentration of the produced sugar, or the like may be appropriately selected. Examples of the post-treatment include solid-liquid separation, ion exchange resin, and freeze drying. The post-treatment may also be a sterilization treatment.

When the formose reaction product contains at least one sugar among formaldehyde (C1a), glycolaldehyde (C2a), glyceraldehyde (C3a), dihydroxyacetone (C3k), erythrulose (C4k), and erythrose and threose (C4a), examples of the post-treatment include a treatment of reducing the content of the sugar in the culture broth to a preferable content described in "1-1-3. Content of sugar" above from the viewpoint of improving the growth environment of microorganisms. Such a treatment is not particularly limited, and examples thereof include dilution of the formose reaction product and a treatment capable of proceeding an aldol reaction of C1 to C4 sugars (for example, base treatment). Among these treatments, the treatment capable of proceeding an aldol reaction of C1 to C4 sugars (in particular, base treatment) is preferable from the viewpoint of reducing the concentration of the sugar without diluting the useful substrate. Examples of the base compound to be used for the base treatment include alkali metal hydroxides (NaOH, KOH, etc.) and alkaline earth metal hydroxides (Ca(OH)₂, Mg(OH)₂, Ba(OH)₂, Sr(OH)₂, etc.). In the base treatment, the liquid property (pH) of the formose reaction product can be increased to, for example, 10 to 14, preferably 11 to 13. After the base treatment, it is better to return the liquid property to neutral (specifically, a liquid property at which microorganisms can grow) again.

### 1-2. Microorganism

The type of the microorganism is not particularly limited as long as it is a microorganism that can heterotrophically grow using a sugar as a substrate (that is, a heterotrophic microorganism having a glycolytic pathway), and examples thereof include bacteria, yeast, and microalgae. Examples of the bacteria include actinomycetes such as Corynebacterium bacteria (for example, Corynebacterium glutamicum), the genus Ralstonia, and the genus Escherichia (for example, Escherichia coli). Examples of the yeast include budding yeast (for example, Saccharomyces cerevisiae), and examples of the microalgae include the genus Spirulina, the genus Chlorella, and the genus Euglena (for example, Euglena gracilis).

As the microorganism, a wild strain may be used, or a mutant strain or a transformant with a mutant gene introduced therein may be used. When a mutant strain or a transformant with a mutant gene introduced therein is used as a microorganism, the type of mutation is also not particularly limited. Examples of the type of mutation include mutation containing a gene designed to produce a substance different from the substance produced by the wild type and/or a gene designed to improve the substance production efficiency.

These mutations may be appropriately selected by those skilled in the art on the basis of the type and/or production efficiency of the target compound (organic compound) set in the production method of the present invention. Technologies relating to mutant microorganisms designed to produce substances different from the substances produced by the wild type are known, and examples thereof include those described in Recent advances in metabolic engineering of Corynebacterium glutamicum for bioproduction of value-added aromatic chemicals and natural products, Applied Microbiology and Biotechnology, October 2018, Volume 102, Issue 20, pp 8685-8705, and the like for mutated Corynebacterium glutamicum; those described in Metabolic engineering of Escherichia coli for direct production of 1,4-butanediol, Nature Chemical Biology, July 2011, Volume 7, Issue 7, pp 445-452, and the like for mutated Escherichia coli; those described in Production of aromatics in Saccharomyces cerevisiae-A feasibility study, Journal of Biotechnology, Volume 163, Issue 2, 20 January 2013, Pages 184-193, and the like for mutated Saccharomyces cerevisiae; and those described in Bioproducts From Euglena gracilis: Synthesis and Applications, Frontiers in Bioengineering and Biotechnology, May 2019, Volume 7, Article 1083, and the like for mutated Euglena gracilis.

The specific concentration of the microorganism in the culture broth is not particularly limited, and examples thereof include 0.5 to 25 w/v%, 5 to 25 w/v%, 10 to 20 w/v%, 12 to 15 w/v%, 0.5 to 20 w/v%, 0.5 to 10 w/v%, 0.5 to 5 w/v%, 0.5 to 3 w/v%, 0.5 to 2 w/v%, and 0.8 to 1.5 w/v%, regardless of the type of microorganism. For example, when the microorganism is a bacterium of the genus Corynebacterium, the concentration is, for example, 0.5 to 25 w/v%, preferably 5 to 25 w/v%, more preferably 10 to 20 w/v%, further preferably 12 to 15 w/v%, and when the microorganism is E. coli, yeast, or microalgae, the concentration is, for example, 0.5 to 25 w/v%, preferably 0.5 to 20 w/v%, 0.5 to 10 w/v%, more preferably 0.5 to 5 w/v%, 0.5 to 3 w/v%, and further preferably 0.5 to 2 w/v%, 0.8 to 1.5 w/v%.

Examples of the specific concentration of the microorganism in the culture broth include 0.5 to 150, 0.5 to 100, 0.5 to 50, 0.5 to 30, 0.5 to 20, 0.5 to 15, 1 to 10, and 2 to 8 in terms of optical density OD600, regardless of the type of the microorganism. For example, when the microorganism is E. coli, yeast, or microalgae, examples of the specific concentration include 0.5 to 50, preferably 0.5 to 30, more preferably 0.5 to 20 and 0.5 to 15, and still more preferably 1 to 10 and 2 to 8.

### 1-3. Substance production conditions

As for the conditions for substance production, the growth conditions of microorganisms can be appropriately determined. Specifically, the production of a substance can be performed under anaerobic conditions, oxygen-limited conditions with trace oxygen, or aerobic conditions, and preferably under anaerobic conditions or oxygen-restricted conditions. For example, when at least one among branched sugars including branched C5 sugars such as 1,3,4,5-tetrahydroxypentan-2-one and 1,3,4-trihydroxy-3-(hydroxymethyl)butan-2-one, and branched C6 sugars such as stereoisomers of 1,2,4,5,6-pentahydroxyhexan-3-one is used as a substrate, and a bacterium of the genus Corynebacterium is used as a microorganism, the production of a substance can be performed under anaerobic conditions or oxygen-restricted conditions. The substance production may be performed in any system among batch, fed-batch, or continuous.

### 1-4. Product (organic compound)

The organic compound to be obtained by the production method of the present invention is not particularly limited as long as it is obtained by substance-converting a substrate by an in vivo reaction of a microorganism, and is determined according to the substrate, the type of the microorganism, and the like. Furthermore, when the microorganism has a mutation, the organic compound is determined according to the design of the mutation.

Examples of the organic compound include organic acids and/or salts thereof, including, for example, lactic acid and/or salts thereof, and the organic compound can also comprise acetic acid, succinic acid, fumaric acid, and/or salts thereof. Examples of the salts include alkali metal salts (for example, sodium salts and potassium salts), and alkaline earth metal salts (for example, calcium salts and magnesium salts). As an example, when bacteria or yeast is used as the microorganism, acetic acid, succinic acid, fumaric acid, and/or salts thereof can be obtained as a product together with lactic acid. Examples of the organic compound include β-glucan, as well. In particular, when the genus Euglena is used as a microorganism, β-glucan can be obtained as a product.

### 2. Cultivation step

The method for producing an organic compound of the present invention preferably comprises a cultivation step of proliferating the microorganism described above in "1-2. Microorganism" before the substance production step. As a result, the microorganism can be subjected to the substance production step in a state where the concentration thereof is sufficiently increased.

In the cultivation step, a cultivation liquid containing a substrate and a microorganism may be incubated under aerobic conditions.

As the substrate to be used for the cultivation of a microorganism, a natural sugar may be used, or a non-natural sugar may be used, or both of them may be used. The types of the natural sugar and the non-natural sugar can be selected from those described in "1-1-1. Sugar contained in sugar-containing liquid" above.

When the substrate to be used for the cultivation of a microorganism contains a natural sugar, preferred examples of the natural sugar include glucose. When the substrate used for the cultivation of a microorganism contains a non-natural sugar, the formose reaction product described in "1-1-4. Example of sugar-containing liquid (sugar-containing liquid containing formose reaction product)" can be used as the substrate.

When the culture broth containing the substrate and the microorganism contains at least one among formaldehyde (C1a), glycolaldehyde (C2a), glyceraldehyde (C3a), dihydroxyacetone (C3k), erythrulose (C4k), and erythrose and threose (C4a) (for example, when a formose reaction product is contained), from the viewpoint of improving the growth rate of the microorganism, the concentration indicated as the concentration of the sugar in the sugar-containing liquid in "1-1-3. Content of sugar" above is preferably adopted as the concentration of the sugar in the culture broth containing the substrate and the microorganism.

The total amount of the sugars in the culture broth containing the substrate and the microorganism is not particularly limited, and is preferably 0.1 to 60 w/v% or 0.5 to 10 w/v%, more preferably 1 to 8 w/v%, still more preferably 3 to 7 w/v%, and further preferably 4 to 6 w/v%.

The medium components contained in the culture broth containing the substrate and the microorganism can be appropriately determined by those skilled in the art according to the growth characteristics of the microorganism, and the like. In addition, the culture broth to be subjected to cultivation may contain any additives as long as the cultivation is not inhibited. Examples of such additives include sugar stabilizers, and more specifically include boric acid, silicic acid, and derivatives thereof. Examples of a derivatization reagent include 2-(hydroxymethyl)phenylboronic acid, 4-formylphenylboronic acid, 4-(methylcarbamoyl)phenylboronic acid, 3-acetamidophenylboronic acid, 2-formylphenylboronic acid, and 2-(dimethylaminomethyl)phenylboronic acid.

### Examples

Hereinafter, the present invention will be described more specifically with reference to examples, but the present invention is not limited thereto. In addition, in the following description, the numerical value of the concentration represented by % means a numerical value represented by w/v% unless otherwise specified.

### (1) Materials used

All materials were used as purchased without purification. 1,3-Dihydroxyacetone dimer, D-erythrose, D-(-)-ribose, D-(-)-lyxose, D-psicose, D-(+)-xylose, D-tagatose, D-(+)-talose, L-(-)-sorbose, D-(+)-allose, L-gulose, D-(+)-mannose, D-(+)-galactose, D-biotin, and 3,4-dihydroxybenzoic acid were obtained from Tokyo Chemical Industry Co., Ltd. Glycolaldehyde dimer, DL-glyceraldehyde, L-(+)-erythrulose, D-ribulose, D-altrose and (2,4-dinitrophenyl)-hydrazine, and yeast nitrogen base (without amino acids) were obtained from Sigma-Aldrich Japan. Formaldehyde solution, D-(-)-arabinose, D-(-)-fructose, D-(+)-glucose, sodium hydroxide, calcium hydroxide, methanol, acetonitrile, phosphoric acid, urea, ammonium sulfate ((NH₄)₂SO₄), potassium dihydrogen phosphate (KH₂PO₄), dipotassium hydrogen phosphate (K₂HPO₄), magnesium sulfate heptahydrate (MgSO₄·7H₂O), iron(II) sulfate heptahydrate (FeSO₄·7H₂O), thiamine hydrochloride, 3-(N-morpholino)propanesulfonic acid (MOPS), calcium chloride dihydrate (CaCl₂-2H2O), zinc sulfate heptahydrate (ZnSO₄-7H₂O), copper(II) sulfate pentahydrate (CuSO₄-5H₂O), nickel(II) chloride hexahydrate (NiCl₂-6H₂O) hydrochloric acid, acetic acid, sulfuric acid, sodium chloride, disodium hydrogen phosphate (Na₂HPO₄), ammonium chloride, Bis-Tris, 4H (EDTA·free acid), sodium lactate, sodium pyruvate, sodium L-(-)-malate, disodium succinate hexahydrate, disodium fumarate, and sodium acetate were purchased from FUJIFILM Wako Pure Chemical Corporation. Phenylhydrazine was obtained from KANTO CHEMICAL CO., INC. Manganese(II) sulfate monohydrate (MnSO₄-H₂O) was obtained from Alfa Aesar. Bacto^{™} Yeast Extract, Bacto^{™} peptone, and Polypeptone^{™} peptone were purchased from Thermo Fisher Scientific. Acid hydrolysate of casein was obtained from Biokar Diagnostics. Deionized water and distilled water were supplied from a Millipore system.

### (2) Instruments used

High-performance liquid chromatography (HPLC) analysis of HCHO and C2-C4 sugars was performed using a Chromaster^{(R)} system equipped with a UV/Vis detector (360 nm; 5430 diode array detector) at a flow rate of 1.0 mL·min⁻¹ using an InertSustain C18 column (150 mm, GL Sciences) and a mixed solvent of wateracetonitrile (6 : 4, v/v) as an eluent.

HPLC analysis of C5-C6 sugars was performed using an HPLC Chromaster^{(R)} Sugar Analysis System (Hitachi, Ltd.) with a fluorescence detector (5440 FL detector) and an NH2P-50 4E column (250 mm, Shodex) as a stationary phase. As mobile phases, solvent A (acetonitrile), solvent B (water), and solvent C (10% aqueous phosphoric acid solution) were used, and the flow rate was set to 1.0 mL·min⁻¹. An aqueous solution of acetic acid (44.3%), phosphoric acid (54.2%), and phenylhydrazine (1.5%) was added to the flow path after the column at a flow rate of 0.4 mL · min⁻¹, and analysis was performed.

In examples in which [I] C. glutamicum was used as a microorganism, for the determination of the amount of an organic acid, operations were performed using an HPLC system (Shimadzu Corporation) equipped with an electrical conductivity detector (CDD-10AVP) and three Shim-pack Fast-OA columns (Shimadzu Corporation) and using a 2.75 mM H₂SO₄ mobile phase at a flow rate of 0.8 mL·min⁻¹ and a column temperature of 50°C. The total organic carbon amount was determined using TOC-L (Shimadzu Corporation).

In examples in which [II] E. coli and [III] S. cerevisiae were used as microorganisms, for the determination of the amount of an organic acid, operations were performed using an HPLC system (Shimadzu Corporation) equipped with an electrical conductivity detector (CDD-10ASP), one TSKgel OApak-P (40 mm, Tosoh), and two TSKgel OApak-A (300 mm, Tosoh) and using a 0.75 mM aqueous H₂SO₄ solution as a mobile phase at a flow rate was 0.7 mL·min⁻¹ and a column temperature of 50°C. An aqueous solution of 0.75 mM H₂SO₄, 20 mM Bis-Tris, and 0.1 mM EDTA-4H was added to the flow path after the columns at a flow rate of 0.7 mL·min⁻¹, and analysis was performed. The total organic carbon amount was determined using TOC-L (Shimadzu Corporation).

### (3) Formose reaction

### (3-1) Preparation of formose reaction product-Ca

The substrates (2M formaldehyde (C1a) and 20 mM dihydroxyacetone (C3k)) were dissolved in a solvent (10% aqueous CH₃OH solution) to form 90 mL of a solution. The solution was placed in a screwtop vial equipped with a stir bar. Before starting a formose reaction, the solution in the vial was heated to 80°C with stirring using an organic synthesis stirrer (HHE-19G-US IV, manufactured by KPI). A catalyst (100 mM Ca(OH)₂) was added to initiate the reaction and the solution was maintained at 80°C for about 1.5 minutes. After the solution became yellow (indicative of the progress of the formose reaction), the vial was cooled with ice-water to stop the reaction. Thereafter, Ca²⁺ was removed using a cation exchange resin Amberlite IR120BHAG (manufactured by Organo Corporation). Unreacted HCHO and CH₃OH were also removed by freeze-drying. The resulting solid powder was re-dissolved in distilled water. The concentrated sugar solution was passed through Amberlite IR120BHAG (manufactured by Organo Corporation) again, whereby the Ca²⁺ cation concentration was lowered. As a result, a formose reaction product -Ca was obtained. The total organic carbon amount was determined using TOC-L (Shimadzu Corporation). As a measurement method, an NPOC (Non-Purgeable Organic Carbon) method was used. The compositions of all monosaccharides produced by the formose reaction are expressed by (CH₂O)ₙ. Therefore, it was assumed that all the constituents of the solution were (CH₂O)ₙ, and the sugar concentration was calculated by the following formula (1).
[Equation 1]

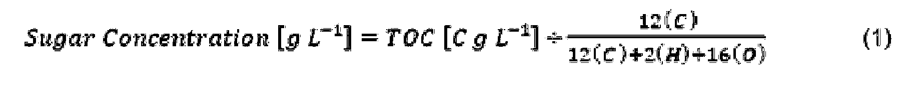

### (3-2) Preparation of formose reaction product-W

A formose reaction product-W was obtained by performing a formose reaction in the same manner as in (3-1) except that sodium tungstate was used as a catalyst in place of 100 mM Ca(OH)₂.

### (4) Derivatization and preparation of analysis

Derivatization for HPLC analysis of HCHO and C2-C4 sugars was performed as follows. The solution (2.5 µL) obtained after the reaction was diluted by adding water (748 µL) thereto. 375 µL of 2,4-dinitrophenylhydrazine (DNPH) in acetonitrile (1 mg·mL⁻¹) and 22.5 µL of an aqueous phosphoric acid solution (20 vol%) were added dropwise to each diluted sample. Each mixed solution was stirred at room temperature for 60 min before HPLC analysis. A sample for HPLC analysis of C5 to C6 sugars was prepared by adding water (250 µL) and acetonitrile (500 µL) to 250 µL of the reaction mixture.

### (5) Phenol-sulfuric acid method

First, 1.5 g of phenol was dissolved in 48.5 mL of ultrapure water to form a 3% phenol solution. Next, 100 µL (10 to 100 µg/mL) of a sample and 200 µL of the 3% phenol solution were added to a test tube and stirred, and then 750 µL of concentrated sulfuric acid was added and stirred, and then the resulting mixture was allowed to stand for 20 minutes. The absorbances of the solution at 490 nm and 480 nm were measured, and the concentrations of a C6 sugar and a C5 sugar were estimated, respectively. For the calibration curves of the C6 sugar and the C5 sugar, glucose and xylose were used as standard substances, respectively.

### (6) Cultivation of microorganism using synthetic sugar

### (6-[1]) Cultivation of C. glutamicum ATCC 13032 strain (wild strain)

A glycerol stock of C. glutamicum ATCC 13032 strain (wild strain) was inoculated into 5 mL of medium A (in 1 L, 2 g of urea, 2 g of yeast extract, 7 g of casamino acid, 7 g of (NH₄)₂SO₄, 0.5 g of KH₂PO₄, 0.5 g of MgSO₄·7H₂O, 6 mg of FeSO₄·7H₂O, 4.2 mg of MnSO₄·H₂O, 0.2 mg of biotin, 0.2 mg of thiamine, and 4% (w/v) glucose were contained), and subjected to shaking cultivation at 30°C, whereby a seed culture broth was prepared. This main culture broth was centrifuged at 3300 xg and 25°C for 5 minutes, and the supernatant was removed.

For a growth experiment of C. glutamicum, a minimal medium CGXII was modified (mCGXII), and (NH₄)2SO₄ (5 g L⁻¹), urea (5 g L⁻¹), KH₂PO₄ (1 g L⁻¹), K₂HPO₄ (1 g L⁻¹), MgSO₄·7H₂O (0.25 g L⁻¹), CaCl₂ (10 mg L⁻¹), FeSO₄-7H₂O (10 mg L⁻¹), MnSO₄-H₂O (0.1 mg L⁻¹), ZnSO₄-7H₂O (1 mg L⁻¹), CuSO₄-5H₂O (0.2 mg L⁻¹), NiCl₂-6H₂O (0.02 mg L⁻¹), biotin (0.2 mg L⁻¹), 3,4-dihydroxybenzoic acid, and MOPS (21 g L⁻¹) were added. The pH of the medium was adjusted to pH 7 with NaOH.

The formose reaction product-Ca obtained in the above (3-1) was filtersterilized, and added to the mCGXII medium at various sugar concentrations (0.5%, 1%, 2%, 3%, 4%, and 5%). Further, wet cells were added to the mCGXII medium containing the formose reaction product and cultivated at 30°C with stirring using a 12 well plate. Separately, in place of the formose reaction product-Ca, various concentrations of each of the C1-C4 sugars (specifically, formaldehyde (C1a), glycolaldehyde (C2a), glyceraldehyde (C3a), dihydroxyacetone (C3k), erythrose (C4a), or erythrulose (C4k)) were used together with 0.1% glucose, and cultivation was carried out in the same manner. The growth of microorganisms was confirmed by measuring the absorbance at 600 nm using a plate reader (Infinite F Nano+, TECAN). The obtained absorbance data was converted to an optical density (OD₆₀₀) at 600 nm. After the cultivation, the sample was filtered to afford a supernatant, which was further analyzed.

### (6-[II]) Cultivation of E. coli K-12 strain (wild strain)

A glycerol stock of E. coli K-12 strain (wild strain) was inoculated into 5 mL of LB medium (in 1 L, 10 g Polypepton, 5 g yeast extract, 10 g sodium chloride, 0.2 mg thiamine, and 4% (w/v) glucose were contained), and subjected to shaking cultivation at 37°C, whereby a seed culture broth was prepared. This main culture broth was centrifuged at 3300 xg and 25°C for 5 minutes, and the supernatant was removed.

The formose reaction product-Ca obtained in the above (3-1) was filtersterilized, and added to a diluted 1.25-fold M9 medium (a medium obtained by adding 1 M MgSO₄ (125 µL), 1 M CaCl₂ (12.5 µL), 1% thiamine (125 µL), and ultrapure water (37.2375 mL) to 12.5 mL of 5-fold M9 medium) prepared by diluting 5-fold M9 medium (in 200 mL, 12 g of Na₂HPO₄, 6 g of KH₂PO₄, 1 g of NaCl, and 2 g NH₄Cl were contained). Further, wet cells were added to the 1.25-fold M9 medium containing the formose reaction product and cultivated at 37°C with stirring using a 96 well plate. The growth of microorganisms was confirmed by measuring the absorbance at 600 nm using a plate reader (Infinite F Nano+, TECAN). The obtained absorbance data was converted to an optical density (OD₆₀₀) at 600 nm. After the cultivation, the sample was filtered to afford a supernatant, which was further analyzed.

### (6-[III]) Cultivation of Yeast strain BY21391 (wild strain)

A glycerol stock of Yeast strain BY21391 (wild strain) was inoculated into 5 mL of a YPD medium (in 1 L, 10 g yeast extract, 20 g peptone, and 20 g glucose were contained), and subjected to shaking cultivation at 30°C, whereby a seed culture broth was prepared. This main culture broth was centrifuged at 3300 xg and 25°C for 5 minutes, and the supernatant was removed.

The formose reaction product-Ca obtained in the above (3-1) was filtersterilized, and added to a YNB medium (in 1 L, 6.7 g of YNB was contained). Further, wet cells were added to the YNB medium containing the formose reaction product and cultivated at 37°C with stirring using a 96 well plate. The growth of microorganisms was confirmed by measuring the absorbance at 600 nm using a plate reader (Infinite F Nano+, TECAN). The obtained absorbance data was converted to an optical density (OD₆₀₀) at 600 nm.

### (7) Production of organic compounds from synthetic sugar using microbial cells

### (7-1) Cultivation step

(7-1-[I])
A glycerol stock of C. glutamicum ATCC 13032 strain was inoculated into 25 mL of a BA medium containing 4% of glucose, and subjected to shaking cultivation at 33°C, whereby a seed culture broth was prepared. This seed culture broth was inoculated into 200 mL of a BA medium containing 4% of glucose, and subjected to shaking cultivation overnight at 33°C in a 500 mL flask. This main culture broth was centrifuged 3 times at 5,500 xg and 4°C for 15 minutes using a BS buffer solution, and the supernatant was removed.

(7 -1-[II])
A glycerol stock of E. coli K-12 strain was inoculated into 40 mL of an LB medium containing 4% of glucose and 0.002% of thiamine, and subjected to shaking cultivation overnight at 37°C. This main culture broth was centrifuged 3 times at 3,300 xg and 4°C for 5 minutes using an M9 medium, and the supernatant was removed.

(7 -1-[III])
A glycerol stock of Yeast strain BY21391 was inoculated into 5 mL of a YPD medium containing 4% of glucose, and subjected to shaking cultivation at 30°C, whereby a seed culture broth was prepared. This seed culture broth was inoculated into 10 mL of a YBD medium containing 4% of glucose, and subjected to shaking cultivation overnight at 30°C. This main culture broth was centrifuged using a YNB medium once at 3,300 xg and 4°C for 5 minutes and twice at 1,800 xg and 4°C for 5 minutes, and the supernatant was removed.

(7-1-[IV])
A subcultivation stock of E. gracilis strain was inoculated into an appropriate amount of a KH medium containing 1.2% of glucose, and subjected to shaking cultivation at 25°C. Thereafter, the mixture was centrifuged at 800 xg for 3 minutes using a 50 mL centrifuge tube, and the supernatant containing the KH medium component was removed. Next, cells were suspended in a CM medium and cultivated. Thereafter, this main culture broth was centrifuged at 800 xg for 3 minutes, and the supernatant was removed.

### (7-2) Substance production step

(7-2-[I])
The wet cells prepared in the above (7-1-[I]) were suspended in a BS buffer solution such that the cell concentration was 12% (w/v). A liquid obtained by filter-sterilizing the formose reaction product-Ca obtained in the above (3-1) or the formose reaction product-W obtained in the above (3-2) was added to the bacterial cell suspension such that the final concentration was 1% (w/v) or 2% (w/v). This solution was placed in a screwtop vial equipped with a stir bar. The vial was maintained at 33°C with stirring under an oxygen-limited condition with trace oxygen using an organic synthesis stirrer (HHE-19G-US IV, KPI), whereby lactic acid fermentation was forced to proceed. After the reaction, the sample was filtered to afford a supernatant, which was quantitatively analyzed with an HPLC equipped with an electrical conductivity detector.

(7 -2-[II])
The wet cells prepared in the above (7-1-[II]) were suspended in an M9 medium such that the cell concentration was 1% (w/v) and the optical density (OD₆₀₀) of the cells was 8. A liquid obtained by filter-sterilizing the formose reaction product-Ca obtained in the above (3-1) or the formose reaction product-W obtained in the above (3-2) was added to the bacterial cell suspension such that the final concentration was 1% (w/v). This solution was placed in a screwtop vial. The vial was maintained at 37°C while being shaken in a thermostatic incubator under an oxygen-limited condition with trace oxygen produced by Ar gas replacement, whereby organic acid fermentation was made to proceed. After the reaction, the sample was filtered to afford a supernatant, which was quantitatively analyzed with an HPLC equipped with an electrical conductivity detector.

(7-2-[III])
The wet cells prepared in the above (7-1-[III]) were suspended in a YNB medium such that the cell concentration was 1% (w/v) and the optical density (OD₆₀₀) of the cells was 8. A liquid obtained by filter-sterilizing the formose reaction product-Ca obtained in the above (3-1) or the formose reaction product-W obtained in the above (3-2) was added to the bacterial cell suspension such that the final concentration was 1% (w/v). This solution was placed in a screwtop vial. The vial was maintained at 37°C while being shaken in a thermostatic incubator under an oxygen-limited condition with trace oxygen produced by Ar gas replacement, whereby organic acid fermentation was made to proceed. After the reaction, the sample was filtered to afford a supernatant, which was quantitatively analyzed with an HPLC equipped with an electrical conductivity detector.

(7-2-[IV])
The wet cells prepared in the above (7-1-[IV]) were suspended in a nitrogendeficient CM medium such that the cell concentration was 1% (w/v) and the optical density (OD₆₀₀) of the cells was 2. A liquid obtained by filter-sterilizing the formose reaction product-Ca obtained in the above (3-1) was added to the bacterial cell suspension such that the final concentration was 1% (w/v). 1300 µL of this solution was added to a 5 mL test tube, and cultivated at a shaking speed of 240 rpm and a temperature of 25°C. After the reaction, β-glucan was extracted and quantitatively analyzed by a phenol-sulfuric acid method.

### (8) Results

### (8-1) Composition of formose reaction product

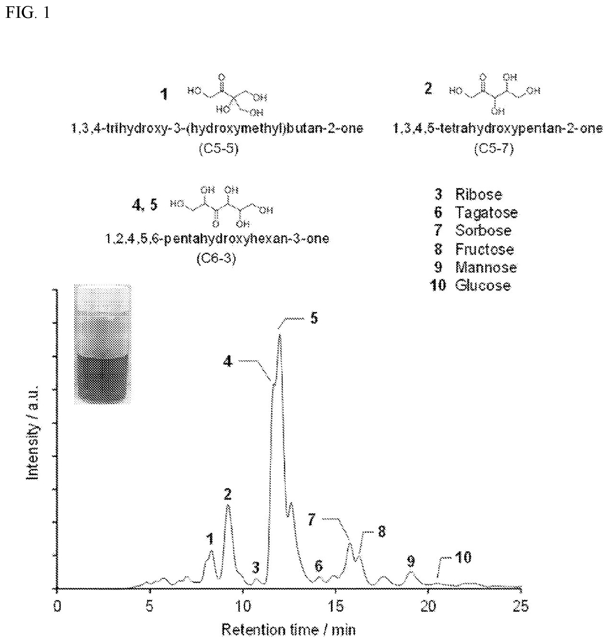

A chromatogram by high-performance liquid chromatography (HPLC) analysis of the formose reaction product-Ca obtained in the above (3-1) is shown in Fig. 1. As shown in Fig. 1, production of linear sugars (specifically, ribose (3), sorbose (7), and fructose (8), etc.) and branched sugars (specifically, 1,3,4-trihydroxy-3-(hydroxymethyl)butan-2-one (1), and stereoisomers of 1,2,4,5,6-pentahydroxyhexan-3-one (3-hexulose (4), (5))) was confirmed.

The concentrations of the respective sugars including the C5-C6 sugars (measured by the phenol-sulfuric acid method), the C2-C4 monosaccharides (specifically, glycolaldehyde (C2a), glyceraldehyde (C3a), dihydroxyacetone (C3k), erythrose (C4a), and erythrulose (C4k)), and the unreacted formaldehyde (C1a) are shown in Table 1.

**[Table 1]**

| | Concentration / mM | Carbon ratio / % |
|---|---|---|
| Formaldehyde **(C1a)** | 2.13 | 0.01 |
| Glycolaldehyde **(C2a)** | 4.01 | 0.05 |
| Glyceraldehyde **(C3a)** | 2.32 | 0.05 |
| Dihydroxyacetone **(C3k)** | 39.01 | 0.76 |
| Erythrose **(C4a)** | 73.92 | 1.92 |
| Erythrulose **(C4k)** | 227.1 | 5.88 |
| C5 sugar | 279.26 | 9.04 |
| C6 sugar | 649.32 | 25.23 |

### (8-2-[I]) Cultivation of C. glutamicum using formose reaction product

### (8-2-[1]-1) Substrate

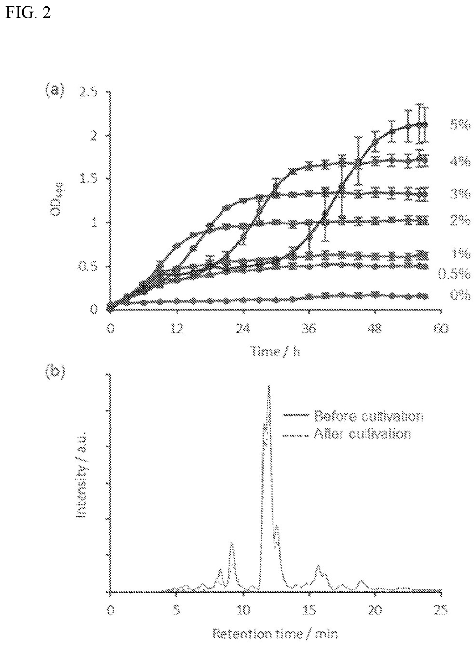

The changes with time of OD₆₀₀ when the formose reaction product was given as a substrate to C. glutamicum at various sugar concentrations (0.5%, 1%, 2%, 3%, 4%, and 5%) in the above (6) are shown in Fig. 2(a). The concentrations shown in Fig. 2(a) were calculated on the basis of the total organic carbon (TOC) value of the synthesized sugar. The OD₆₀₀ increased only when the formose reaction product was added, whereas no increase in the OD₆₀₀ value was observed when the formose reaction product was not added. The final OD₆₀₀ value increased with an increase in the addition amount of the formose reaction product. These results indicate that the synthetic sugars contained in the formose reaction products served as substrates for C. glutamicum.

The HPLC analysis results of the culture broth supernatants before the start of cultivation and 57 hours after the start of cultivation, in the case where 0.5% of the synthetic sugar liquid was added are shown in Fig. 2(b). As shown in Fig. 2(b), it has been confirmed that even when the synthetic sugar liquid having the lowest concentration shown in Fig. 2(a) was used, the peak intensities of some sugar components decreased after cultivation. The decreased peaks are attributed to linear sugars (specifically, 1,3,4,5-tetrahydroxypentan-2-one (2), ribose (3), and fructose (8)), and branched sugars (specifically, 1,3,4-trihydroxy-3-(hydroxymethyl)butan-2-one (1) and stereoisomers of 1,2,4,5,6-pentahydroxyhexan-3-one (3-hexulose (4), (5)). Thus, it has been found that wild-type cells of C. glutamicum can metabolize several synthetic sugars including branched sugars (particularly non-natural sugars).

### (8-2-[I]-2) Control of growth rate of microorganisms

### Control factor

The influence of a monosaccharide with a short carbon chain (that is, an intermediate of a formose reaction) in the synthetic sugar liquid on the growth was evaluated. Table 2 shows the concentrations of the respective C1-C4 sugars when the sugar concentration of the formose reaction solution-Ca obtained in the above (3-1) was 5%.

**[Table 2]**

| | Concentration / mM (sugar 5%) |
|---|---|
| Formaldehyde **(C1a)** | 0.23 |
| Glycolaldehyde **(C2a)** | 0.43 |
| Glyceraldehyde **(C3a)** | 0.25 |
| Dihydroxyacetone **(C3k)** | 4.21 |
| Erythrose **(C4a)** | 7.97 |
| Erythrulose **(C4k)** | 24.49 |

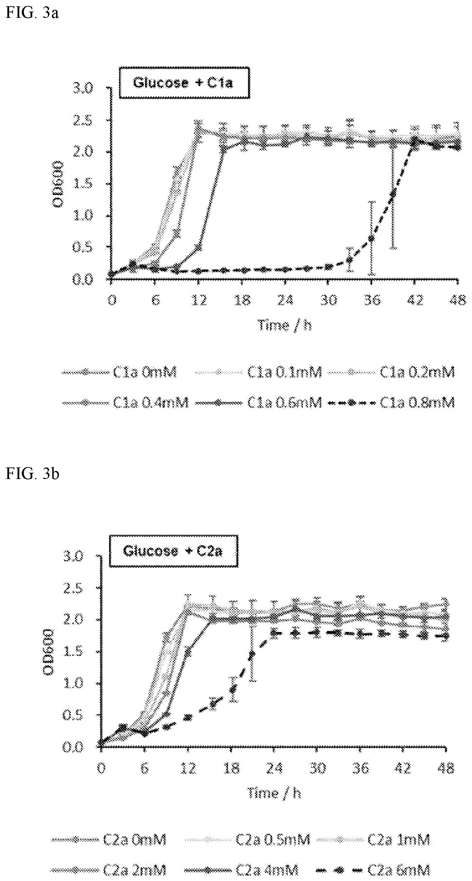

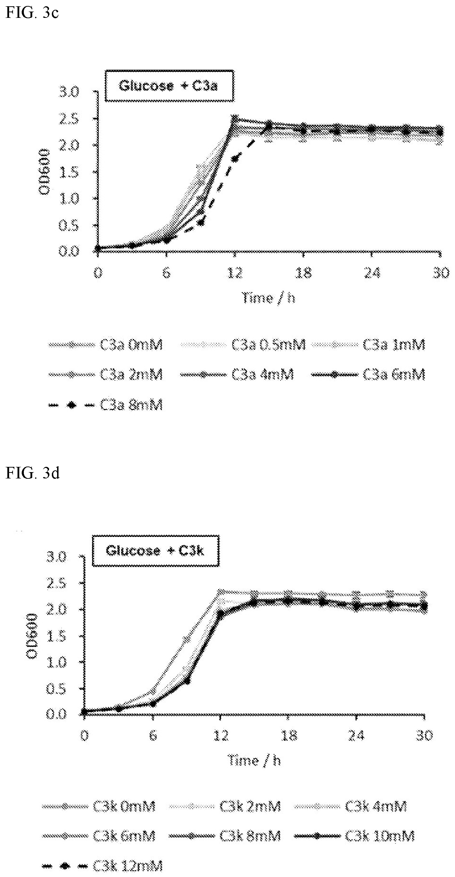

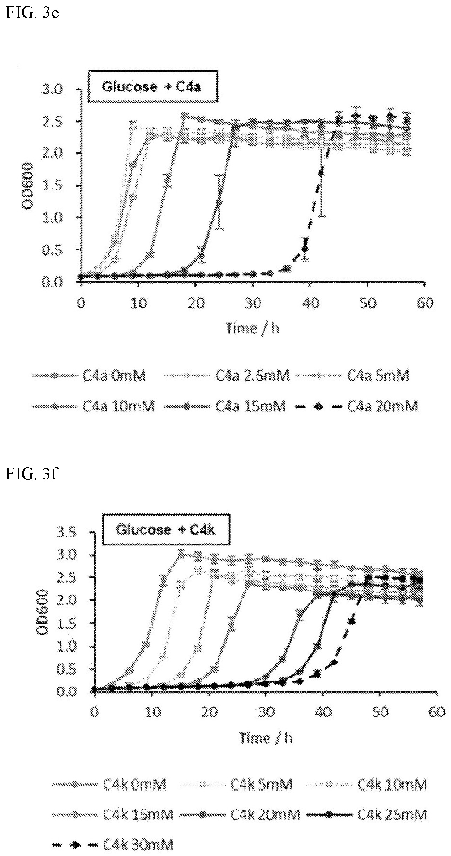

On the other hand, the changes in OD₆₀₀ with time when the C1-C4 sugars described above were each added at different concentrations in the above (6) together with 0.1% of glucose instead of the formose reaction product are shown in Figs. 3a to 3f. As shown in Figs. 3a to 3f, it has been revealed that in the growth of microorganisms, C1 to C4 sugars serve as control factors of the growth rate, and the growth rate of the microorganisms can be increased by reducing the amount of coexistence of these sugars. That is, reducing the amount of the coexisting C1 to C4 sugars provides a growth environment desirable for microorganisms, and not only can be applied as a desirable condition in a case where microorganisms are grown using a non-natural sugar-containing liquid such as a formose reaction product, but also can be reasonably inferred to be applied as a desirable condition in a case where substances are produced using microorganisms in the non-natural sugar-containing liquid.

### Treatment to control growth rate of microorganisms

NaOH was added to the formose reaction product-Ca obtained in the above (3-1) to increase the pH to 12, and the resulting mixture was allowed to stand at room temperature overnight, then the pH was returned to neutrality, and quantitative HPLC analysis was performed. The compositions of the synthetic sugar liquid before and after the base treatment are shown in Table 3.

**[Table 3]**

| | Concentration / mM Before base treatment (sugar 5%) | Concentration / mM After base treatment |
|---|---|---|
| Formaldehyde **(C1a)** | 0.23 | 0.08 |
| Glycolaldehyde **(C2a)** | 0.43 | 0.1 |
| Glyceraldehyde **(C3a)** | 0.25 | 0.12 |
| Dihydroxyacetone **(C3k)** | 4.21 | 0.27 |
| Erythrose **(C4a)** | 7.97 | 2.62 |
| Erythrulose **(C4k)** | 24.49 | 4.96 |

As shown in Table 3, the C1 to C4 sugars were each decreased by the base treatment of the formose reaction product. This is considered to be because the aldol reactions of the C1 to C4 sugars, which are formose intermediate products, were made to proceed by the function of the base catalyst.

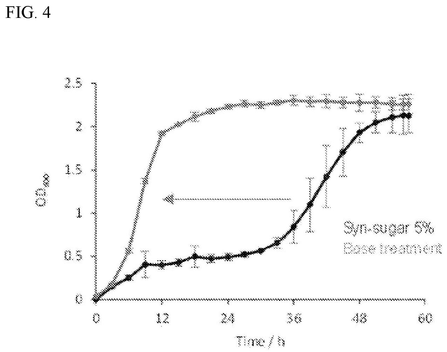

In particular,C4k was decreased to about 1/5 (4.96 mM) by the base treatment though it was contained at a relatively high ratio in the formose reaction product-Ca obtained by the above (3-1). According to the results of Fig. 3f, when the C4k concentration is 5 mM or less, the growth rate is increased remarkably as much as the delay in the growth rate can be ignored when compared to the case of using glucose alone as a substrate. Therefore, when the base-treated formose reaction product is used, the growth rate can be expected to be remarkably increased. To confirm this, the change in OD₆₀₀ with time when the base-treated formose reaction product was actually given to cells instead of the formose reaction product (before base treatment) in the above (6) is shown in Fig. 4. As shown in Fig. 4, when the base-treated formose reaction product was used as a substrate, the cell growth rate could be remarkably increased. That is, the base treatment of the formose reaction product provides a growth environment desirable for microorganisms, and not only can be applied as a desirable condition when microorganisms are grown using the formose reaction product but also can be reasonably inferred to be applied as a desirable condition when a substance is produced using the microorganisms in a sugar-containing liquid containing the formose reaction product.

### (8-2-[II]) Cultivation of E. coli using formose reaction product

The findings on the substrate and the findings on the control of the growth rate of microorganisms were similar to the findings obtained in the above "(8-2-[I]) Cultivation of C. glutamicum using formose reaction product".

### (8-2-[III]) Cultivation of yeast using formose reaction product

The findings on the substrate and the findings on the control of the growth rate of microorganisms were similar to the findings obtained in the above "(8-2-[I]) Cultivation of C. glutamicum using formose reaction product".

### (8-2-[IV]) Cultivation of E. gracilis using formose reaction product

The findings on the substrate and the findings on the control of the growth rate of microorganisms were similar to the findings obtained in the above "(8-2-[I]) Cultivation of C. glutamicum using formose reaction product".

### (8-3) Substance production using formose reaction product

### (8-3-[I]) Substance production by C. glutamicum using formose reaction product

The HPLC chromatogram of the supernatant after the reaction (24 hours) obtained by substance production using 1 w/v% of the formose reaction product-Ca in the above (7) is shown in Fig. 5(a) together with the HPLC chromatogram of the supernatant immediately after the start of the reaction (0 hours). As shown in Fig. 5(a), consumption of C5-C6 sugars similar to the results of the cell growth in Fig. 2(b) was observed. The HPLC chromatogram obtained by performing the same operations except that no cells were added in the above (7) is shown in Fig. 5(b). As shown in Fig. 5(b), when cells were not contained, a decrease in peak intensity was not observed.

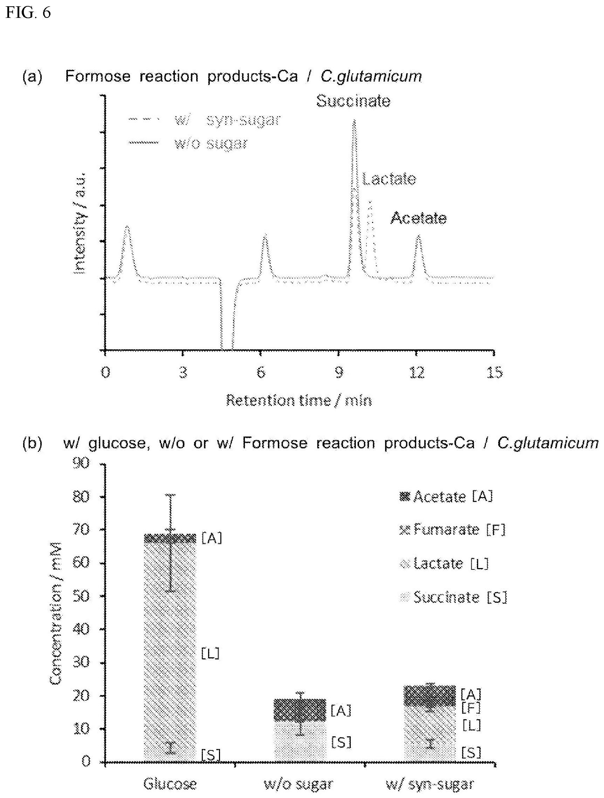

Further, the result of the HPLC analysis of the reaction product obtained by the substance production using 1 w/v% of the formose reaction product-Ca in the above (7) is shown in Fig. 6 (a). As shown in Fig. 6(a), when the formose reaction product was added (w/syn-sugar), lactic acid was produced in synchronization with the decrease of C5-C6 sugars, and such lactic acid production was not confirmed when no sugar was added (w/o sugar).

On the basis of the result of Fig. 6(a), the amounts of the lactic acid and other organic acids obtained by the substance production using 1 w/v% of the formose reaction product-Ca were determined on the basis of the difference in concentration between immediately after the start of the reaction (0 hour) and after the reaction (24 hours). The results are shown in Fig. 6(b). In Fig. 6(b), in addition to the case where the formose reaction product was added as a substrate (w/syn-sugar) and the case where no sugar was added (w/o sugar), the results of the case where only glucose (1.5%) was added as a substrate (Glucose) are also shown.

As shown in Fig. 6(b), from glucose alone (Glucose), production of lactic acid (62 mM) as a main product and small amounts of succinic acid (4.1 mM) and acetic acid (2.9 mM) was also confirmed. The amount of glucose consumed for 24 hours corresponded to 45% of the initial amount, and the yields of lactic acid relative to the initial amount and the consumption of glucose were about 33% and about 83%, respectively. Also when no sugar was added (w/o sugar), succinic acid (12 mM) and acetic acid (6.7 mM) were produced. These organic acids under the w/o sugar condition may be derived from organics accumulated in advance in the cells. On the other hand, when the formose reaction product-Ca was added (w/syn-sugar), about 11 mM of lactic acid was produced, and the lactic acid yield from 1% (w/v) of the formose reaction product-Ca was 10.2%. In addition, by-products such as succinic acid (5.5 mM), acetic acid (6.2 mM), and a small amount of fumaric acid (0.04 mM) were also confirmed.

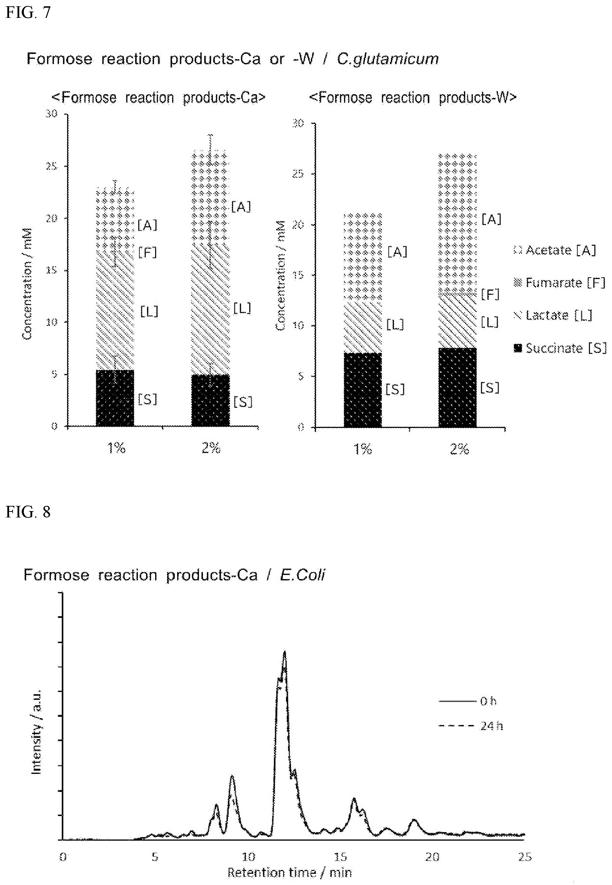

Similarly to Fig. 6(b), the result of the determination of the amounts of the organic acids obtained by substance production using 2 w/v% of the formose reaction product-Ca in the above (7), and the result of the determination of the amounts of the organic acids obtained by substance production using 1 w/v% or 2 w/v% of the formose reaction product-W are shown in Fig. 7 in the same manner as in Fig. 6(b). In Fig. 7, the result of the determination of the amounts of the organic acids obtained by w/syn-sugar (the substance production using 1 w/v% of the formose reaction product-Ca) shown in Fig. 6(b) is also shown again.

For the case of w/syn-sugar (the substance production using 1 w/v% of the formose reaction product-Ca) in Fig. 6(b), the concentrations of C1 to C4 sugars before and after the lactic acid fermentation reaction are shown in Table 4.

**[Table 4]**

| | Concentration / mM Before the reaction | Concentration / mM After the reaction |
|---|---|---|
| Formaldehyde **(C1a)** | 0.06 | 0.06 |
| Glycolaldehyde **(C2a)** | 0.08 | 0.02 |
| Glyceraldehyde **(C3a)** | 0.11 | 0.04 |
| Dihydroxyacetone **(C3k)** | 0.54 | 0.03 |
| Erythrose **(C4a)** | 1.18 | 0.5 |
| Erythrulose **(C4k)** | 3.82 | 1.22 |

As shown in Table 4, the concentrations of C3k, C4k, and other sugars also had decreased at the time after the lactic acid fermentation. This fact suggests that these sugars, as well as sugar alcohols and sugar carboxylic acids whose amounts were not measured, partially served as a carbon source for the lactic acid production.

### (8-3-[II]) Substance production by E. Coli using formose reaction product

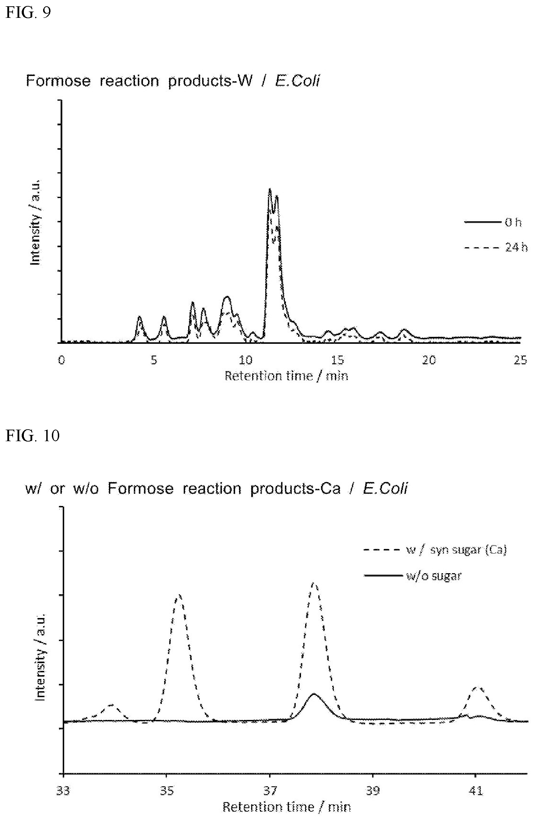

The HPLC chromatogram of the supernatant after the reaction (24 hours) obtained by substance production using 1 w/v% of the formose reaction product-Ca in the above (7) is shown in Fig. 8 together with the HPLC chromatogram of the supernatant immediately after the start of the reaction (0 hours). Similarly, the HPLC chromatogram of the supernatant after the reaction (24 hours) obtained by substance production using 1 w/v% of the formose reaction product-W in the above (7) is shown in Fig. 9 together with the HPLC chromatogram of the supernatant immediately after the start of the reaction (0 hours). As shown in Figs. 8 and 9, consumption of C5-C6 sugars was observed.

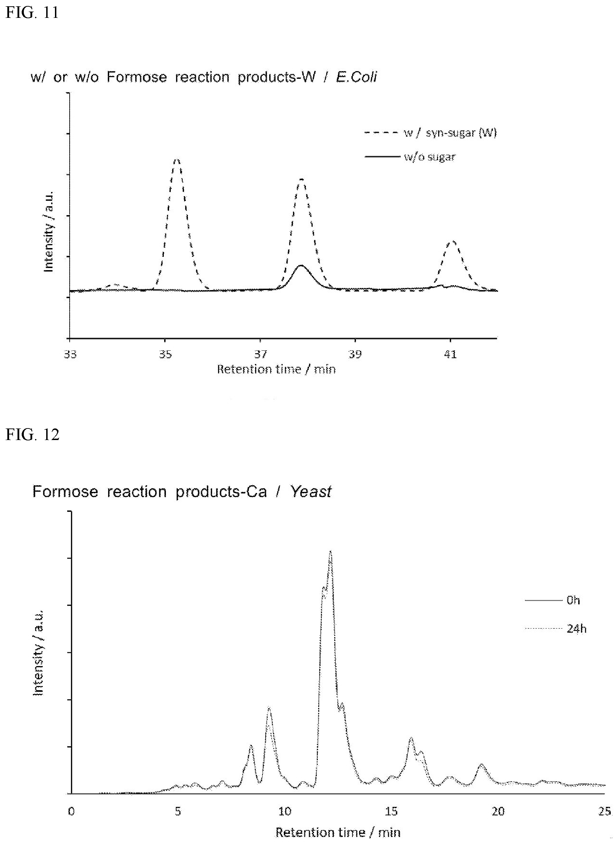

Further, the result of the HPLC analysis of the reaction product obtained by the substance production using 1 w/v% of the formose reaction product-Ca in the above (7) is shown as (w/syn-sugar) in Fig. 10, and the result of the HPLC analysis of the reaction product obtained by the substance production using 1 w/v% of the formose reaction product-W in the above (7) is shown as (w/syn-sugar) in Fig. 11. In both figures, the result of the HPLC analysis of the reaction product obtained by performing the same operations except that the formose reaction product-Ca was not used is also shown as (w/o sugar). As shown in Figs. 10 and 11, when the formose reaction product was added (w/syn-sugar), lactic acid was produced in synchronization with the decrease of C5-C6 sugars, and such lactic acid production was not confirmed when no sugar was added (w/o sugar).

### (8-3-[III]) Substance production by Yeast using formose reaction product

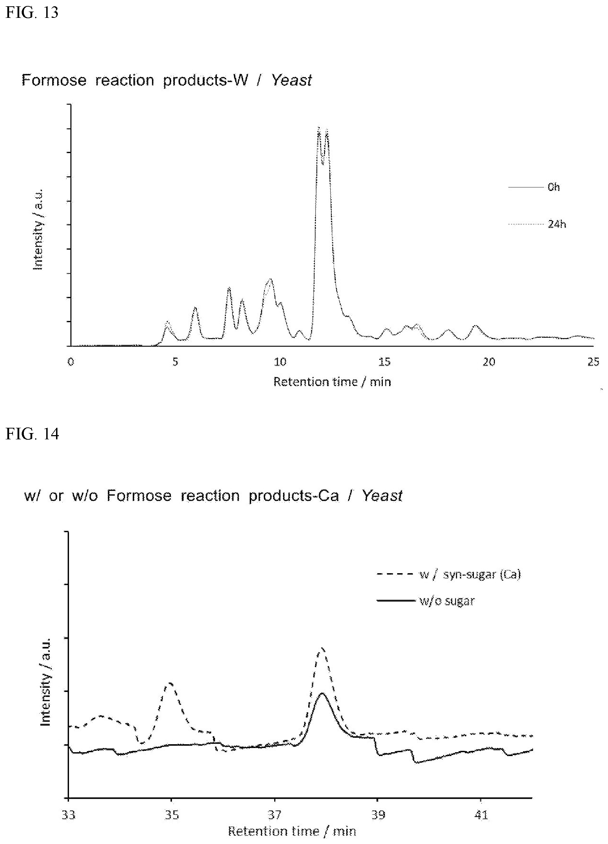

The HPLC chromatogram of the supernatant after the reaction (24 hours) obtained by substance production using 1 w/v% of the formose reaction product-Ca in the above (7) is shown in Fig. 12 together with the HPLC chromatogram of the supernatant immediately after the start of the reaction (0 hours). Similarly, the HPLC chromatogram of the supernatant after the reaction (24 hours) obtained by substance production using 1 w/v% of the formose reaction product-W in the above (7) is shown in Fig. 13 together with the HPLC chromatogram of the supernatant immediately after the start of the reaction (0 hours). As shown in Figs. 12 and 13, consumption of C5-C6 sugars was observed.

Further, the result of the HPLC analysis of the reaction product obtained by the substance production using 1 w/v% of the formose reaction product-Ca in the above (7) is shown as (w/syn-sugar) in Fig. 14, and the result of the HPLC analysis of the reaction product obtained by the substance production using 1 w/v% of the formose reaction product-W in the above (7) is shown as (w/syn-sugar) in Fig. 15. In both figures, the result of the HPLC analysis of the reaction product obtained by performing the same operations except that the formose reaction product-Ca was not used is also shown as (w/o sugar). As shown in Figs. 14 and 15, when the formose reaction product was added (w/syn-sugar), organic acids such as acetic acid were produced in synchronization with the decrease of C5-C6 sugars.

### (8-3-[IV]) Substance production by E. gracilis using formose reaction product

The concentration (concentration in terms of a C1 sugar) of the C2-C4 sugars contained in the supernatant after the reaction (24 hours) obtained by substance production using 1 w/v% of the formose reaction product-Ca in the above (7) is shown in Fig. 16(a) together with the concentration (concentration in terms of a C1 sugar) of the C2-C4 sugars contained in the supernatant immediately after the start of the reaction (0 hours). As shown in Fig. 16(a), consumption of C2-C4 sugars was observed.

Further, the concentration (concentration in terms of a C1 sugar) of the β-glucan contained in the reaction product obtained by the substance production using 1 w/v% of the formose reaction product-Ca in the above (7) is shown as (w/ sugar) in Fig. 16(b) together with the concentration (concentration in terms of a C1 sugar) of the β-glucan contained in the reaction product obtained by performing the same operations except that the formose reaction product-Ca was not used as (w/o sugar). As shown in Fig. 16(b), production of β-glucan was observed.

### (9) Conclusion

The coryneform bacteria and E. coli shown in the above test examples are heterotrophic prokaryotes, yeast is a heterotrophic eukaryote, and euglena is a heterotrophic and autotrophic eukaryote. As shown in the above test examples, it has been confirmed that organic compounds such as organic acids and β-glucan can be produced from non-natural sugars in a wide range of species. In general, since the metabolism of sugars in vivo is commonly performed in a glycolytic pathway, organic acids are considered to be products obtained by metabolizing non-natural sugars in a glycolytic pathway. In addition, β-glucan is considered to be a product via a paramylon (β-1,3-glucan) synthesis pathway in the genus Euglena. That is, it can be reasonably inferred that even microbial species different from those described above can produce substances via an in vivo pathway such as a glycolytic pathway or a paramylon synthesis pathway.

## Claims

1. A method for producing an organic compound, the method comprising a substance production step for, in a sugar-containing liquid containing a non-natural sugar, causing a microorganism to produce a substance by using the non-natural sugar as a substrate.

2. The production method according to claim 1, further comprising a step of cultivation of the microorganism before the substance production step.

3. The production method according to claim 1, wherein a concentration of the microorganism in the sugar-containing liquid is 0.5 to 25 w/v%.

4. The production method according to claim 1, wherein the non-natural sugar comprises at least one among a triose through a hexose.

5. The production method according to claim 1, wherein the non-natural sugar is selected from the group consisting of stereoisomers of 1,3,4,5-tetrahydroxypentan-2-one, 1,3,4-trihydroxy-3-(hydroxymethyl)butan-2-one, and 1,2,4,5,6-pentahydroxyhexan-3-one.

6. The production method according to claim 1, wherein the sugar-containing liquid contains at least one among formaldehyde (C1a), glycolaldehyde (C2a), glyceraldehyde (C3a), dihydroxyacetone (C3k), erythrulose (C4k), and erythrose (C4a) and threose (C4a).

7. The production method according to claim 1, wherein the sugar-containing liquid contains erythrulose (C4k) at an amount to be more than 0 mM and not more than 20 mM.

8. The production method according to claim 1, wherein the sugar-containing liquid contains erythrose and/or threose (C4a) at an amount to be more than 0 mM and not more than 25 mM in total.

9. The production method according to claim 1, wherein the sugar-containing liquid contains formaldehyde (C1a) at an amount to be more than 0 mM and not more than 0.6 mM, glycolaldehyde (C2a) at an amount to be more than 0 mM and not more than 4 mM, glyceraldehyde (C3a) at an amount to be more than 0 mM and not more than 7 mM, and/or dihydroxyacetone (C3k) at an amount to be more than 0 mM and not more than 4 mM.

10. The production method according to claim 1, wherein the sugar-containing liquid contains a formose reaction product.

11. The production method according to claim 1, wherein the sugar-containing liquid contains a base-treated product of a formose reaction product.

12. The production method according to claim 1, wherein the sugar in the sugar-containing liquid is contained at an amount of 0.1 to 60 w/v% in total.

13. The production method according to claim 1, wherein the organic compound comprises lactic acid and/or a salt thereof.

14. The production method according to claim 1, wherein the organic compound comprises acetic acid, succinic acid, fumaric acid, and/or salts thereof.

15. The production method according to claim 1, wherein the organic compound comprises β-glucan.

16. The production method according to claim 1, wherein the microorganism is selected from the group consisting of bacteria, yeast, and microalgae.
